# EUROPEAN PATENT SPECIFICATION

(11) **EP 2 040 778 B1**
(45) Date of publication and mention of the grant of the patent: **21.09.2011**
(21) Application number: 07870723.9
(22) Date of filing: 12.07.2007
(51) Int. Cl.: A61M 5/178, G21F 5/018

(54) **RADIATION SHIELDED SYRINGE ASSEMBLY**
SPRITZENANORDNUNG MIT STRAHLUNGSABSCHIRMUNG
ENSEMBLE DE SERINGUE BLINDÉE CONTRE LES RAYONNEMENTS ET SES UTILISATIONS

(30) Priority: 19.07.2006 US 831761 P
(43) Date of publication of application: 01.04.2009
(73) Proprietor: Mallinckrodt LLC, Hazelwood, MO 63042 (US)
(72) Inventor: BRUCE, John K., Burlington, KY 41005 (US); FAGO, Frank M., Mason, OH 45040 (US)
(74) Representative: Chettle, Adrian John
(86) International application number: PCT/US2007/015948
(87) International publication number: WO 2008/076150

(56) References cited:
- EP-A- 0 661 066
- US-A- 3 973 554
- US-A- 4 056 096
- US-A- 6 159 144
- US-A1- 2006 086 909

## Description

### FIELD OF THE INVENTION

The present invention relates generally to syringes and, more particularly, to radiation shielding for syringes used with radioactive materials, such as radiopharmaceuticals.

### BACKGROUND

This section is intended to introduce the reader to various aspects of art that may be related to various aspects of the present invention, which are described and/or claimed below. This discussion is believed to be helpful in providing the reader with background information to facilitate a better understanding of the various aspects of the present invention. Accordingly, it should be understood that these statements are to be read in this light, and not as admissions of prior art.

Nuclear medicine utilizes radioactive material for diagnostic and therapeutic purposes by injecting a patient with a small dose of the radioactive material, which concentrates in certain organs or biological regions of the patient. Radioactive materials typically used for nuclear medicine include Technetium-99m, Indium-113m, and Strontium-87m among others. Some radioactive materials naturally concentrate toward a particular tissue, for example, iodine concentrates toward the thyroid. However, radioactive materials are often combined with a tagging or organ-seeking agent, which targets the radioactive material for the desired organ or biologic region of the patient. These radioactive materials alone or in combination with a tagging agent are typically referred to as radiopharmaceuticals in the field of nuclear medicine. At relatively lower doses of the radiopharmaceutical, a radiation imaging system (e.g., a gamma camera) provides an image of the organ or biological region that collects the radiopharmaceutical. Irregularities in the image are often indicative of a pathologic condition, such as cancer. Higher doses of the radiopharmaceutical may be used to deliver a therapeutic dose of radiation directly to the pathologic tissue, such as cancer cells.

In certain applications, such as nuclear medicine, a syringe may intake, contain, and subsequently inject a radioactive substance, such as a radiopharmaceutical. Unfortunately, some radiation exposure may occur while filling, transporting, and administering the radioactive material in the syringe. Existing syringes generally do not provide radiation shielding throughout these various stages of filling, transporting, administering, and generally handling the syringe filled with radioactive material. Each of these various stages involves at least some radiation exposure. In some applications, multiple independent syringes may inject various substances, such as radioactive material, tagging or organ seeking agents, biocompatible flushing substances, and so forth. Unfortunately, the timing, number, and other perimeters of these multiple injections may increase the likelihood or duration of radiation exposure.

US-A-2004/0015038 discloses a syringe shield having the features of the pre-characterizing portion of claim 1 appended hereto.

### SUMMARY

The present invention, in certain embodiments, relates to a radiation shield that may be disposed about a syringe in a manner that permits operation of the syringe, while still providing radiation shielding to contain radioactivity from a radiopharmaceutical disposed in the syringe. A radiation shield of the invention may be associated with (placed about) a syringe, used to shield a technician during an injection procedure that includes the syringe, dissociated from the syringe, and subsequently reused with another syringe for another injection. In other words, the radiation shield of at least some embodiments may be described as independent or removable from the syringe. As a result, the radiation shield may be used with a conventional disposable syringe, which can substantially reduce the costs associated with providing radiation shielding for a syringe.

Certain embodiments commensurate in scope with the originally claimed invention are set forth below. It should be understood that these embodiments are presented merely to provide the reader with a brief summary of certain forms the invention might take and that these embodiments are not intended to limit the scope of the invention. Indeed, the invention may encompass a variety of features and embodiments that may not be set forth below.

A first aspect of the present invention relates to a radiation shield for use with a syringe. This radiation shield includes a sleeve and a cover, each having radiation shielding material in their makeup. The sleeve and the cover may be removably coupled with another to substantially enclose a syringe within the radiation shield. Incidentally, the phrase "removably coupled" herein refers to a relationship of at least first and second structures, whereby the structures are brought together (e.g., linked or connected) in a manner enabling easy subsequent dissociation of the structures from each other. The sleeve of the radiation shield has a syringe receptacle defined therein to accommodate a portion of a syringe. In addition, the cover has a syringe end receptade and a passage defined therein. The syringe end receptacle may be utilized to accommodate at least an end portion of the syringe, while the passage may be utilized to accommodate a nozzle or tip of the syringe. As such, the passage defined in the cover of the radiation shield may allow fluid to be drawn into and/or expelled out of the nozzle of the syringe while the radiation shielding material is located about the syringe.

A radiopharmaceutical system that includes a syringe and a syringe radiation shield is disclosed herein. The syringe includes a barrel having a first end and a second end opposite from the first end, and a plunger movably disposed inside and along the barrel. The syringe radiation shield includes a first sleeve, a second sleeve, and an end cover, all having radiation shielding to shield a user from radiation being emitted from radioactive material within the syringe. The first sleeve may be disposed about the barrel, and the second sleeve may be disposed at least partially concentric with the first sleeve. In context of the first and second sleeves, the phrase "at least partially concentric" may be defined as at least a portion of the length of the first sleeve is concentric or coaxial with at least a portion of the length of the second sleeve. The second sleeve is disposed about and generally movable with the plunger. Similarly, the end cover may be removably coupled with and at least partially concentric with the first sleeve. Again, in context of the end cover and the first sleeve, the phrase "at least partially concentric" may be defined as at least a portion of the length of the cover is concentric or coaxial with at least a portion of the length of the first sleeve.

A syringe that includes a barrel having a first end and a second end opposite from the first end is disclosed herein. A plunger of the syringe is movably disposed in the barrel relative to the first end, and a valve core extends outwardly from the second end of the barrel. The valve core may be utilized to generally control fluid flow by opening and closing one or more fluid passages, for example, in response to a change in position of a surrounding structure. For example, the valve core may include a central passage leading to a lateral passage, and the surrounding structure may include a cap. The cap may be disposed about the valve core and may be movable between an open position and a sealed position with the valve core. In other words, the cap may be positioned to either cover or uncover the lateral passage in the valve core. Thus, the syringe itself (e.g., via the valve core) can control the fluid flow through the second end of the barrel.

A method of using a radiation shielded syringe assembly is disclosed herein. In this method, fluid flow in a syringe of the assembly is controlled, at least in part, by moving a first radiation shielded member of the assembly relative to a second radiation shielded member of the assembly. At least a portion of the first radiation shielded member is disposed about the syringe. For example, movement of the first radiation shielded member may induce movement of a plunger of the syringe assembly. In such an embodiment, the first and second radiation shielded members may include first and second concentric sleeves, wherein the first sleeve moves lengthwise along the second sleeve to enable movement of the plunger. By further example, movement of the first radiation member may induce movement of a valve. In such an embodiment, the first and second radiation shielded members may include a cap and a sleeve, wherein the cap moves relative to the sleeve to open and close a passage in the syringe.

Various refinements exist of the features noted above in relation to the various aspects of the present invention. Further features may be incorporated in these various aspects as well. These refinements and additional features may exist individually or in any combination. For instance, various features discussed below in relation to one or more of the illustrated embodiments may be incorporated into any of the above-described aspects of the present invention alone or in any combination. Again, the brief summary presented above is intended only to familiarize the reader with certain aspects and contexts of the present invention without limitation to the claimed subject matter.

### BRIEF DESCRIPTION OF THE FIGURES

These and other features, aspects, and advantages of the present invention will become better understood when the following detailed description is read with reference to the accompanying figures in which like characters represent like parts throughout the figures, wherein:

FIG.1 is an exploded, cross-sectional view of a radiation shielded syringe assembly;

FIG. 2 is a cross-sectional view of the radiation shielded syringe assembly of FIG. 1;

FIG. 3 is a partial, cross-sectional view of the radiation shielded syringe assembly of FIGS. 2, illustrating a removable tip shielding system in a no flow configuration;

FIG. 4 is a partial, cross-sectional view of the radiation shielded syringe assembly of FIG. 3, illustrating the removable tip shielding system in a flow configuration;

FIG. 5 is an exploded, cross-sectional view of another radiation shielded syringe assembly;

FIG. 6 is a cross-sectional view of the radiation shielded syringe of FIG. 5;

FIG. 7 is a partial, cross-sectional view of the radiation shielded syringe of FIGS. 5 and 6;

FIG. 8 is a partial, cross-sectional view of the radiation shielded syringe assembly of FIG. 7;

FIG. 9 is an exploded, cross-sectional view of yet another radiation shielded syringe assembly;

FIG. 10 is a cross-sectional view of the radiation shielded syringe assembly of FIG. 9;

FIG. 11 is an exploded, cross-sectional view of still another radiation shielded syringe assembly;

FIG. 12 is a cross-sectional view of the radiation shielded syringe assembly of FIG. 11;

FIG. 13 is a flowchart of a nuclear medicine process;

FIG. 14 is a block diagram of a radiopharmacy system; and

FIG.15 is a block diagram of a nuclear imaging system.

### DETAILED DESCRIPTION OF SPECIFIC EMBODIMENTS

One or more specific embodiments of the present invention will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

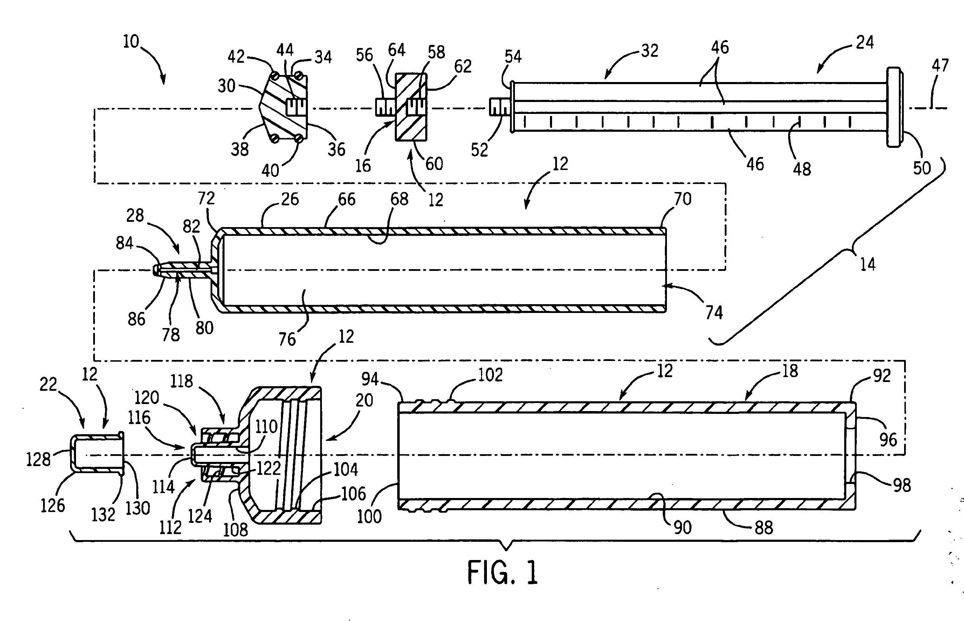

FIG. 1 shows an exemplary embodiment of a radiation shielded syringe assembly 10 including a radiation shield 12 exploded with respect to a syringe 14. As discussed in further detail below, the radiation shield 12 may be utilized with a variety of different syringes, including the syringe 14, to substantially block or reduce the likelihood and/or extent of radiation exposure from a radioactive material (e.g., radiopharmaceutical) disposed within the syringe 14. Various radiation shields of the invention may be designed/configured to be utilized with a variety of conventional syringes, such as disposable plastic syringes. The syringe 14 is shown as being a manual syringe having no electronic, electrical, or motorized parts, such that the syringe 14 is relatively compact, lightweight, and portable to facilitate hand operation. Although the radiation shield 12 may increase the weight and size, the overall syringe assembly 10 may still be substantially more compact, lightweight, and portable in comparison to existing power injectors having motors, electronics, and so forth. In some embodiments, the syringe assembly 10 may be mounted to an automated injection system, which may include electronics, controls, stepper motors, hydraulics, pneumatics, and/or other features to operate the syringe assembly 10 locally or remotely from a control unit.

The radiation shield 12 may be utilized to substantially enclose (e.g., house) the syringe 14 during injection, filling, transport, and/or general handling. After an injection procedure is complete, the syringe 14 may be disposed of (e.g., discarded) by appropriate procedures, while the radiation shield 12 may be reused with another syringe. As such, the radiation shield 12 may reduce costs associated with providing radiation shielding for syringes, while generally reducing the likelihood and/or extent of radiation exposure from a radioactive material disposed within the syringe 14. As discussed in further detail below, the syringe assembly 10 may include a variety of different removable radiation shields, such as the illustrated radiation shield 12 of FIG. 1.

The radiation shield 12 is shown as including a radiation shielded plunger insert 16, a radiation shielded sleeve 18, a radiation shielded cover 20, and a radiation shielded tip fitting 22 (e.g., luer plug). In general, these components 16, 18, 20, and 22 of the radiation shield 12 may include a variety of radiation shielding materials, such as lead, depleted uranium, tungsten, tungsten impregnated plastic, and/or the like. Regarding manufacturing techniques, these components 16, 18, 20, and 22 may be formed by any appropriate techniques such as molding, extruding, machining, and various combinations thereof. However, the manufacturing techniques and materials for these components 16, 18, 20, and 22 are not limited to these particular examples. In addition to the radiation shield 12, the illustrated syringe 14 of the assembly 10 may include a variety of single fluid injection or multi-fluid injection features. For example, the illustrated syringe 14 includes a plunger 24 and an elongated syringe barrel 26 having what may be characterized as a flow control tip 28 at one end thereof. This flow control tip 28 of the syringe barrel 26 is configured to cooperate with a portion of the radiation shield 12 to control fluid flow of the syringe 14. Other embodiments of the flow control tip may exhibit other manners of controlling fluid flow from the syringe 14.

The plunger 24 of the syringe 14 includes a plunger head 30 coupled to a push rod 32. The plunger head 30 includes a generally cylindrical body 34 having a flat side 36 and an opposite tapered (e.g., conical) side 38. A plurality of seals (e.g., o-rings 40, 42) is disposed about the body 34 of the plunger head 30. In certain embodiments, the o-rings 40 and 42 may be formed of rubber or another resilient or flexible material, while the body 34 may be formed of a plastic or other generally rigid material. The plunger head 30 is also shown as having a fastening mechanism 44 associated therewith, for example, for coupling the plunger head 30 with the pushrod 32. In the illustrated embodiment, the fastening mechanism 44 refers to female threads extending into the body 34 of the plunger head 30 at a central position in the flat side 36 thereof. However, other embodiments may include other appropriate fastening mechanisms.

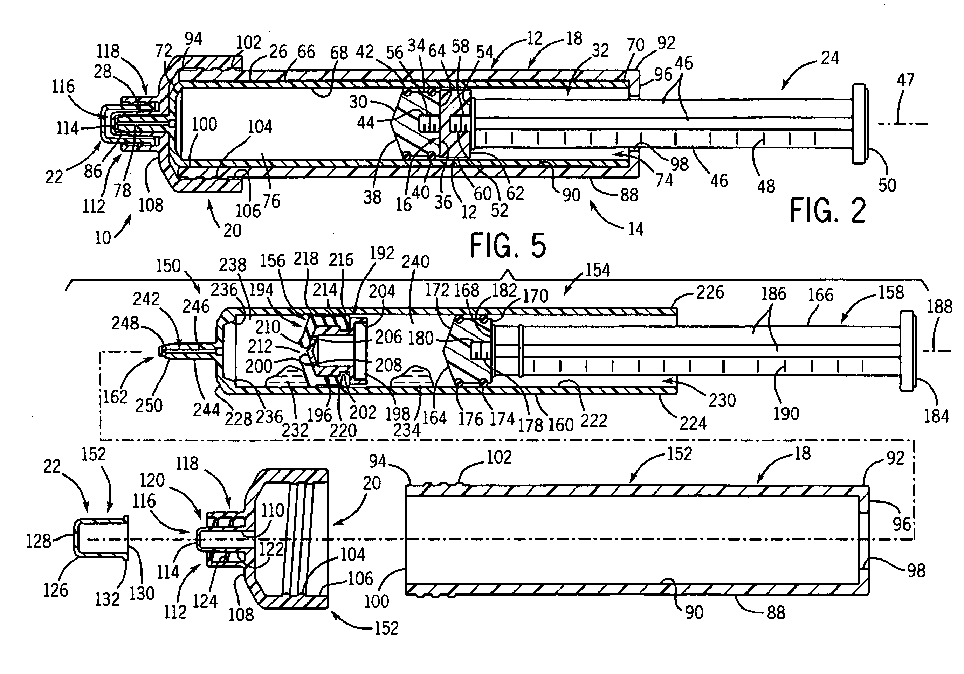

The push rod 32 of the syringe 14 may be formed of plastic or another appropriate material. The illustrated push rod 32 includes lengthwise ribs 46. More particularly, the push rod 32 includes four ribs symmetrically arranged about a lengthwise, central axis 47 (Fig. 2) of the assembly 10. The push rod 32 may include measurement indicia 48 disposed in a sequentially offset arrangement lengthwise along one or more of the ribs 46. The push rod 32 is shown as having an end member 50 (e.g., a thumb tab) at one end thereof and a fastening mechanism 52 disposed at an opposite side 54. This fastening mechanism 52 may be cooperatively designed to fasten together with the fastening mechanism 44 of the plunger head 30. For example, in the illustrated embodiment, the fastening mechanism 52 is shown as an externally threaded member (e.g., male threads) configured to be screwed into the cooperatively designed female threads of the plunger head 30. Other embodiments may exhibit other appropriate designs of the fastening mechanism 52.

The insert 16 of the radiation shield 12 may be disposed removably between the plunger head 30 and the push rod 32 and fastened to the same using the fastening mechanisms 44, 52 of the plunger head 30 and push rod 32 (respectively). For example, the insert 16 is shown as having fastening mechanisms 56, 58 associated therewith. In particular, these fastening mechanisms 56, 58 are shown as an externally threaded member and an internally threaded member. It is generally preferred that the fastening mechanisms 56, 58 be configured to mate with the corresponding fastening mechanisms 44, 52 of the plunger head 30 and the push rod 32 (respectively). Similar to the plunger head 30, the insert 16 may include a body 60 having opposing sides 62, 64.

The body 60 of the insert 16 may be removably coupled to the plunger head 30 by rotatingly driving the male threads of the fastening mechanism 56 into the female threads of the fastening mechanism 44 of the plunger head 30, such that the side 64 of the insert 16 may eventually interface with the side 36 of the plunger head 30 (e.g., in a generally flush manner). Similarly, the push rod 32 may be removably coupled with the body 60 of the insert 16 by rotatingly driving the male threads of the fasting mechanism 52 into the corresponding female threads of the fastening mechanism 58 of the body 60, such that the side 54 of the push rod 32 may eventually interface with the side 62 of the body 60 (e.g., in a substantially flush manner). The insert 16 may be removed by unthreading both the plunger head 30 and the push rod 32 from the fastening mechanisms 56, 58. As such, the insert 16 may be installed onto a plunger 24 utilized in a radiopharmaceutical procedure, and removed from the plunger 24 to be utilized again in another future radiopharmaceutical procedure. Incidentally, while one embodiment of the insert 16 is shown, it should be noted that other embodiments may be utilized with a variety of syringes having fastening mechanisms between the plunger head and the push rod thereof. For instance, the insert 16 of some embodiments may be utilized with syringes having and/or may include fastening mechanisms, such as press fit mechanisms, latches, snap-fit mechanisms, luer fittings, and so forth.

The syringe barrel 26 of the syringe 14 is shown as having a generally cylindrical exterior surface 66 and a corresponding cylindrical interior surface 68 defining a generally cylindrical passage extending lengthwise along the central axis 47 between a first end 70 and a second end 72 of the syringe barrel 26. The first end 70 has an opening 74 defined therein that enables the plunger 24 and its components to pass independently or collectively into and out of the syringe barrel 26. For example, the plunger head 30 may be coupled with the insert 16 and inserted into the syringe barrel 26 through the opening 74 independent from the push rod 32, which may be subsequently installed (e.g., after assembly of the sleeve 18 and the cover 20 about the syringe barrel 26).

The o-rings 40, 42 and the body 60 of the insert 16 may be generally flush with the interior 68 of the syringe barrel 26. As such, the o-rings 40, 42 may substantially provide a fluid seal within the syringe barrel 26 while the insert 16 may substantially block radiation or reduce the likelihood and/or extent of radiation exposure from the fluid 76 in a direction along the central axis 47 out through opening 74. In combination, the insert 16 and the other radiation shielded components 18, 20, and 22 may substantially or entirely contain radiation or reduce the likelihood of radiation exposure from the fluid 76 in all directions from areas within the syringe barrel 26. In other words, the radiation shielded components 16, 18, 20, and 22 may substantially surround and block radiation from all sides or portions of the syringe 14. The fluid 76 may include and/or refer to a radiopharmaceutical or radioisotope, such as technetium-99m.

The flow control tip 28 at the second end 72 of the syringe barrel 26 may include a variety of internal and external geometries to facilitate flow control of the fluid 76 disposed within the syringe 14. For example, the flow control tip 28 may include a passage 78 dispose within a nozzle 80. This passage 78 may include a central passage 82 leading from the interior 68 of the syringe barrel 26 to a downstream set of one or more lateral passages 84 disposed in a generally tapered end 86 (e.g., conical tip portion) of the flow control tip 28. These lateral passages 84 and the tapered end 86 may removably interface or cooperate with portions of the cover 20 to selectively seal and/or unseal the lateral passages 84, thereby selectively enabling or disabling flow of the fluid 76 through the flow control tip 28.

Turning again to the radiation shield 12, the remaining radiation shielded components 18, 20, and 22 may be removably assembled about or retrofitted with the syringe barrel 26 to provide additional radiation shielding in combination with the insert 16. As illustrated in FIG. 1, the sleeve 18 may include a generally cylindrical exterior 88 and a corresponding cylindrical interior 90 disposed lengthwise along the majority of the sleeve 18 between a first end 92 and an opposite second end 94. In addition, the sleeve 18 may include a flange 96 (e.g., syringe abutment end) having a first opening 98 (e.g., plunger passageway) at the first end 92. Similarly, the second end 94 may include a second opening 100. The sleeve 18 may include a removable fastening mechanism, such as external threads 102, disposed at or near the second end 94. The cover 20 may include a removable fastening mechanism, such as internal threads 104, disposed along a generally cylindrical interior 106. Accordingly, the cover 20 may be removably coupled to the sleeve 18 by rotatingly driving the internal threads 104 about the external threads 102. However, a variety of other fastening mechanisms, such as snap-fittings, latches, press fittings, and various tool-free or tool-less fasteners (i.e., fasten without the use of tools), may be used to couple together the sleeve 18 and the cover 20.

The illustrated cover 20 may include a syringe abutment end or substantially closed front 108 having a central passage 110 extending lengthwise through an external fluid coupling 112 (e.g., end fitting) from the interior 106 to a final fluid outlet 114. In certain embodiments, the end fitting 112 may include a luer fitting or another medical coupling mechanism. For example, the illustrated end fitting 112 may include a male portion 116 (e.g., male luer) and a supplemental securement portion 118 (e.g., luer collar). For example, the luer collar 118 may be disposed concentrically about the male luer 116, such that these components 116 and 118 define a receptacle or interspace 120 having one or more removable fastening mechanisms. By further example, the male luer 116 may include a compression fitting or tapered or external surface 122, while the luer collar 118 may include internal threads 124. In certain embodiments, the end fitting 112 may include a flow control mechanism to open and close the fluid flow relative to the syringe 14. For example, the flow control mechanism may operate by moving (e.g., threading or unthreading) the cover 20 inwardly and outwardly relative to the sleeve 18, or by covering or generally sealing the flow control tip 28 of the syringe 14, or by pinching the flow control tip 28, or by blocking the flow control tip 28 (e.g., via the male luer 116 and/or the luer plug 22), or a combination thereof. By further example, the valve actuator or flow control mechanism of the end fitting 112 may include the fluid outlet 114 of the passage 110 and a movable fastening mechanism (e.g., threads 102 and 104) between the sleeve 18 and the cover 20. As discussed below, the flow control tip 28 of the syringe 14 may generally cooperate with the flow control mechanism of the end fitting 112 to open or close fluid flow through the fluid outlet 114.

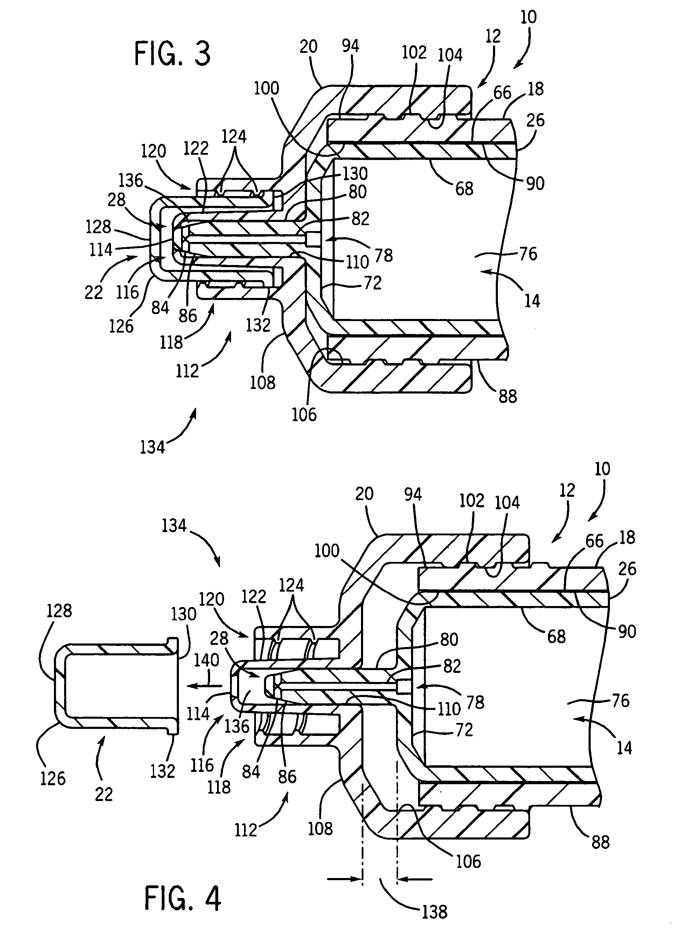

Finally, the luer plug 22 may include a cup shaped body 126 having a closed end 128 and an open end 130 with adjacent fastening members 132, such as opposite luer tabs or external luer threads. Accordingly, the luer plug 22 may be removably coupled to the end fitting 112 of the cover 20 by removably driving the external luer threads 132 into the internal threads 124 about the male luer 116. As such, the luer plug 22 may substantially or completely cover the fluid outlet 114, thereby substantially or completely reducing the likelihood of radiation exposure at the flow control tip 28 of the syringe 14. Moreover, as discussed in further detail below with reference to FIGS. 3 and 4, the radiation shielded components 18, 20, and 22 may cooperate with one another to selectively open or close the lateral passages 84 of the flow control tip 28.

FIG. 2 is a cross-sectional view of an embodiment of the syringe assembly 10 of FIG. 1, further illustrating the radiation shield 12 generally assembled or retrofitted with respect to the syringe 14. Referring now generally to FIGS. 1 and 2, the syringe assembly 10 may be assembled as follows. As discussed above, the plunger head 30 may be removably coupled to the insert 16 independently from the push rod 32. The assembly of the plunger head 30 and the adapter 16 may then be inserted lengthwise into the syringe barrel 26 through the opening 74. At this point, the sleeve 18 may be disposed about the syringe barrel 26 by inserting the first end 70 of the syringe barrel 26 into the second end 94 of the sleeve 18. More specifically, the exterior 66 of the syringe barrel 26 may be moved slidingly along the interior 90 of the sleeve 18 until the first end 70 of the syringe barrel 26 reaches the first end 92 of the sleeve 18. Subsequently, the cover 20 may be removably coupled to the sleeve 18 as discussed in detail above. For example, the cover 20 may be threaded onto the second end 94 of the sleeve 18 by rotatingly driving the internal threads 104 about the external threads 102 until the closed front 108 generally reaches the second end 94.

At this point, the push rod 32 may be assembled with the previously assembled plunger head 30 and insert 16 inside the syringe barrel 26. Specifically, the push rod 32 may be inserted through the first opening 98 of the sleeve 18 until the male threads 52 reach the female threads 58 on the insert 16. Then, the push rod 32 may be rotated to drive the male threads 52 of the push rod 32 into the female threads 58 of the insert 16. In certain embodiments, the push rod 32 may be pushed inwardly until the plunger head 30 abuts the second end 72 of the syringe barrel 26, such that the abutment may hold the plunger head 30 while the push rod 32 is rotated to thread with the insert 16. However, in some embodiments, the plunger head 30 and the insert 16 may include a slot or groove that engages with a lengthwise rib extending along the interior 68 of the syringe barrel 26, such that the engagement between the groove and the rib may rotatingly secure the plunger head 30 and the insert 16 during the threaded engagement with the push rod 32. Finally, the luer plug 22 may be coupled to the end fitting 112 as discussed above.

The fully assembled or retrofitted syringe assembly 10 is illustrated in FIG. 2. Again, the various components 16,18, 20, and 22 of the radiation shield 12 may substantially or entirely block or contain the radiation originating with the fluid 76 inside the syringe 14. Accordingly, the syringe assembly 10 may be safely transported, handled, or generally used with a lower likelihood of radiation exposure. In certain embodiments, the radiation shield 12 may further include a radiation shielded sleeve disposed about the length of the push rod 32, such that the additional radiation shielded sleeve provides radiation shielding continuously at the first opening 98 as the push rod 32 slides inwardly and outwardly along the syringe barrel 26. Alternatively, the push rod 32 may be replaced with a radiation shielded push rod, e.g., a hollow cylindrical or solid cylindrical shaft.

FIG. 3 is a partial cross-sectional view of the syringe assembly 10 of FIG. 2, further illustrating a removable tip shielding system 134 in a no flow configuration. Specifically, the removable tip shielding system 134 may include the flow control tip 28 of the syringe barrel 26, the sleeve 18, the cover 20, and the luer plug 22. As illustrated in FIGS. 2 and 3, the cover 20 is threadingly coupled to the sleeve 18, such that the syringe barrel 26 is generally secured or compressively held between the closed front 108 of the cover 20 and the flange 96 of the sleeve 18.

In certain embodiments, the first end 70 or the second end 72 of the syringe barrel 26 may be fastened or generally coupled to the first end 92 or the second end 94 of the sleeve 18. For example, the first end 70 or the second end 72 of the syringe barrel 26 may include external threads, a snap-fit mechanism, a latch mechanism, or another tool free coupling mechanism, while the first end 92 or the second end 94 of the sleeve 18 has a mating fastener or coupling. In the illustrated embodiment of FIGS. 2 and 3, the exterior 66 of the syringe barrel 26 may be generally compressively fit within the interior 90 of the sleeve 18. As such, the compressive fit secures or generally holds the syringe barrel 26 lengthwise within the sleeve 18. The retention of the syringe barrel 26 within the sleeve 18 may facilitate the operation of the removable tip shielding system 134 as discussed in detail below.

In the no flow configuration of the removable tip shielding system 134 of FIG. 3, the cover 20 may be completely threaded onto the sleeve 18, such that the second end 72 of the syringe barrel 26 abuts the closed front 108 of the cover 20 and the flow control tip 28 extends fully into the central passage 110 of the end fitting 112. As such, the flow control tip 28 may be generally sealed within the central passage 110 of the male luer 116. Specifically, the tapered end 86 of the nozzle 80 may extend at least against, into, or through the fluid outlet 114 of the male luer 116. As a result, the tapered end 86 may be at least substantially or completely sealed against the fluid outlet 114, while the lateral passages 84 may remain upstream from the fluid outlet 114 in a fluid cavity 136. As illustrated in FIG. 3, the fluid cavity 136 may be generally closed off or sealed by the interface between the male luer 116 and the nozzle 80 of the flow control tip 28. Thus, any fluid disposed within the passage 78 may be substantially blocked or restricted within the fluid cavity 136, such that the fluid may not spill, leak, or generally escape from the syringe assembly 10. Accordingly, the system 134 may reduce the likelihood of radiation exposure associated with fluid leakage of a radioactive fluid 76, such as a radiopharmaceutical, disposed within the syringe 14.

In addition, the luer plug 22 may supplement the sealed interface between the male luer 116 and flow control tip 128. As illustrated in FIG. 3, the luer plug 22 may be removably coupled to the end fitting 112 via engagement of the threads 124 and 132, thereby removably securing the cup shaped body 126 over the flow control tip 28 and the fluid outlet 114. As such, the luer plug 22 may provide additional radiation shielding that may reduce the likelihood of radiation exposure if any radioactive fluid 76 escapes from the flow control tip 28 and the male luer 116.

FIG. 4 is a partial cross-sectional view of an embodiment of the radiation shielded syringe of FIGS. 2 and 3, further illustrating the removable tip shielding system 134 disposed in a flow configuration. Specifically, as illustrated in FIG. 4, the luer plug 22 may be unthreaded and released from the end fitting 112. In addition, the cover 20 may be partially but not completely unthreaded from the sleeve 18, thereby creating an interspace, gap, or general offset 138 between the second end 72 of the syringe barrel 26 and the closed front 108 of the cover 20. As a result, the offset 138 translates to an equal gap or offset between the flow control tip 28 and the fluid outlet 114 of the male luer 116. In other words, the offset 138 causes the fluid cavity 136 to expand and open adjacent the passage 78 of the flow control tip 28.

In this open or flow configuration, the syringe assembly 10 may be operated to output, input, or generally exchange one or more substances, such as the fluid 76, with respect to the interior 68 of the syringe 14. For example, the plunger 24 may be depressed inwardly along the lengthwise/central axis 47 of FIGS. 1 and 2, such that the fluid 76 flows into and through the flow control tip 28. As illustrated in FIG. 4, the flow configuration of the system 134 may enable the fluid 76 to pass lengthwise along the central passage 82, outwardly through the lateral passages 84, into the fluid cavity 136, and then outwardly from the fluid outlet 114 as indicated by arrow 140. Similarly, the plunger 24 may be pulled or moved outwardly from the system 134 lengthwise along the central axis 47 of FIGS. 1 and 2, thereby drawing, suctioning, or generally intaking one or more fluids into the interior 68 via the flow control tip 28. More specifically, the fluid may enter through the fluid outlet 114, pass through the fluid cavity 136, enter the passage 78, and fill the interior 68 between the second end 72 and the plunger head 30 of the syringe 14. In certain applications, the forgoing procedure may be used to either fill or discharge the fluid 76, such as a radiopharmaceutical, while the radiation shield 12 may substantially reduce or eliminate the likelihood of radiation exposure. The end fitting 112 may be coupled to a variety of fluid delivery systems, fluid charging systems, or general fluid exchange systems. For example, the end fitting 112 may be coupled to medical tubing, a fluid supply container, a fluid output container, a patient injection system, or various combinations thereof.

FIGS. 5-8 illustrate an alternative embodiment of a radiation shielded syringe assembly 150 including a radiation shield 152 and a syringe 154 (e.g., a multi-chamber or multi-injection syringe). FIG. 5 is an exploded cross-sectional view of an embodiment of the assembly 150, illustrating various features of the radiation shield 152 and the syringe 154 generally exploded from one another. As illustrated in FIG. 5, the radiation shield 152 may include the sleeve 18, the cover 20, and the luer plug 22 as illustrated and described above with reference to FIGS. 1-4. In addition, the radiation shield 152 may include one or more secondary radiation shielded floating valve plungers 156 (e.g., intermediate flow control plungers), which may supplement or replace the insert 16 as illustrated and described above with reference to FIGS. 1-4.

Similar to the radiation shield 12 of FIGS. 1-4, the radiation shield 152 may extend substantially or entirely around one or more radioactive materials disposed within the syringe 154, thereby reducing the likelihood of radiation exposure. Again, the radiation shielded components 18, 20, 22, and 156 may be generally assembled or retrofitted with a variety of different syringes, such as the syringe 154. Moreover, a substantial portion or all of the radiation shielded components 18, 20, 22, and 156 may be removed and reused for a plurality of procedures involving radioactive materials, whereas the syringe, e.g., 154, may be disposable. As a result, the syringe 154 may be a relatively low cost product made of plastic or another suitable material. For these reasons among others, the radiation shield 152 may substantially reduce costs and potential radiation exposure associated with radioactive substances, such as radial pharmaceuticals, used with syringes in various procedures.

In the illustrated embodiment of FIG. 5, the syringe 154 may include a primary plunger 158 and an elongated fluid container 160 (e.g., syringe barrel) having a flow control tip 162. In addition, one or more of the floating plungers 156 may replace or supplement other non-shielded floating plungers of the syringe 154. Similar to the embodiment of FIGS. 1-4, the primary plunger 158 includes a primary plunger head 164 coupled to a push rod 166. For example, the primary plunger head 164 may be removably coupled to the push rod 166 via a variety of fastening mechanisms, such as mating threads, snap-fit mechanisms, compression fit mechanisms, or various tool free fasteners. In the illustrated embodiment, the primary plunger head 164 may include a body 168 having a flat end 170 and an opposite curved or conical side or end 172. In addition, the primary plunger head 164 may include one or more outer seals, such as a plurality of sequential o-rings 174 and 176, disposed about the body 168. The primary plunger head 164 may include a removable fastening mechanism, such as an internally threaded member 178 (e.g., female threads) extending inward from the flat end 170. Similarly, the push rod 166 may include a removable fastening mechanism, such as an externally threaded member 180 (e.g., male threads), extending outwardly from a flat end 182. Thus, the primary plunger head 164 may be removably coupled to the push rod 166 by rotatingly driving the male threads 180 into the female threads 178 until the flat ends 170 and 182 are generally flush with one another. In addition, the push rod 166 may include an end member 184 (e.g., thumb tab) disposed on an opposite end from the male threads 180. Similar to the embodiment of FIGS. 1-4, the push rod 166 may include lengthwise ribs 186, such as four ribs, arranged symmetrically about a lengthwise/central axis 188 of the primary plunger 158. Measurement indicia 190 may be disposed along the length of the pushrod or shaft 166 in a generally sequential offset arrangement.

As mentioned above, the syringe 154 of FIG. 5 may include one or more floating plungers, e.g., 156. In certain embodiments, the floating plunger 156 may include a generally central, internal, or flow through check valve. In other words, the floating plunger 156 may be configured to enable fluid to pass directly through rather than around the floating plunger 156 in response to a pressure differential between opposite sides of the floating plunger 156. In the illustrated embodiment, the floating plunger 156 may include a radiation shielded adapter 192 (e.g., fluid passage insert) and a flexible plunger sleeve 194 (e.g., check valve). For example, the fluid passage insert 192 and the check valve 194 may have generally circular or annular geometries, which may be disposed concentrically with respect to one another.

The illustrated fluid passage insert 192 may include a body portion 196 having a first open end 198 and an opposite second perforated end 200 (e.g., throat portion). In addition, the body portion 196 may include an annular groove 202 and a protruding annular collar 204 (e.g., flange portion) disposed adjacent the first open end 198. The throat portion 200 may have a generally tapered, inwardly angled, or conical geometry, which includes one or more fluid passages. For example, the throat portion 200 may include axially offset passages 206 and 208, which may be normally closed/sealed by the check valve 194. In certain embodiments, the throat portion 200 may include fewer or greater numbers of passages, such as 1, 3, 4, 5, 6, 7, 8, 9, 10, or more. These passages, e.g., 206 and 208, enable fluid to flow directly through the interior of the floating plunger 156, rather than around the periphery of the floating plunger 156 at the seal interface with the syringe barrel 160. As illustrated, the axially offset passages 206 and 208 may be substantially covered and sealed by a flexible perforated face 210 (e.g., mouth portion) of the check valve 194. In other words, the mouth portion 210 may be substantially or mostly closed across the throat portion 200 of the fluid passage insert 192 except for a central axial opening 212. As illustrated, the opening 212 may be disposed along the axis 188, whereas the axially offset passages 206 and 208 may be disposed at a substantial distance or offset from the axis 188.

The check valve 194 includes a body 214 having annular outer seals 216 and 218 (e.g., o-ring portions) and a generally annularly rib 220 (e.g., latch portion). In the illustrated embodiment, the body 214 of the check valve 194 may be disposed concentrically about the body portion 196 of the fluid passage insert 192, such that the latch portion 220 may extend removably into the annular groove 202. As such, the fluid passage insert 192 may be removably coupled or snap-fit with the check valve 194, such that the floating plunger 156 may be disassembled, cleaned, and reused if desirable. In certain embodiments, the fluid passage insert 192 may be molded, machined, or generally manufactured with a variety of radiation shielding materials, such as lead, depleted uranium, tungsten impregnated plastic, and so forth. The check valve 194 may be molded or generally manufactured from a variety of flexible or resilient materials, such as rubber. As discussed in further detail below, the fluid passage insert 192 cooperates with the check valve 194 to substantially or entirely block radiation, control fluid flow, and generally separate fluids disposed on opposite sides of the floating plunger 156. Again, upon reaching or passing a certain pressure differential between opposite sides of the floating plunger 156, the check valve 194 may enable fluid flow directly through an interior of the fluid passage insert 192 rather than around the periphery of the floating plunger 156.

As further illustrated in FIG. 5, the syringe barrel 160 includes a generally cylindrical interior 222 (e.g., passage) and a generally cylindrical exterior 224, which both extend lengthwise along the syringe barrel 160 between a first end 226 (e.g., inlet) and a second end 228 (e.g., outlet). In certain applications, one or more of the floating plungers 156 and the primary plunger 158 may be disposed lengthwise along the interior 222 through a plunger opening 230 at the first end 226. The plungers 156 and 158 may be disposed at an offset from one another and from the second end 228 to accommodate two or more substances or fluids. For example, a first substance 232 (e.g., medical fluid) may be disposed between the floating plunger 156 and the second end 228 of the syringe barrel 160. In addition, a second substance 234 (e.g., medical fluid) may be disposed between the primary plunger head 164 and the floating plunger 156. In certain embodiments, the first substance 232 may include a radiopharmaceutical, a contrast agent, a drug, or a combination thereof. By further example, the second substance 234 may include a biocompatible flushing or cleaning substance, such as a heparin solution, sterilized water, a glucose solution, saline, or another suitable substance. The interspacing between the one or more floating plungers 156, the primary plunger head 164, and the second end 228 of the syringe barrel 160 may depend on the volume, quantity, or dose of the first substance 232, the second substance 234, and so forth.

The syringe barrel 160 may include an offset plunger stop 236 (e.g., annular flow control actuator) extending outwardly between the second end 228 and the interior 222. As discussed in further detail below, the plunger stop 236 may engage the outer periphery of the floating plunger 156, such that the mouth portion 210 may be forced forward away from the throat portion 200 to enable injection or general flow of the second substance 234. In other words, the first substance 232 disposed in a first chamber 238 may be forced outwardly through the flow control tip 162 in response to forward movement of the floating plunger 156. Upon reaching the plunger stop 236, the check valve 194 of the floating plunger 156 opens in a forward direction to enable the second substance 234 disposed in a second chamber 240 to flow directly through the interior of the floating plunger 156 in response to axial movement of the primary plunger 158.

Similar to the embodiment of FIGS. 1-4, the flow control tip 162 of FIG. 5 may include crosswise passages 242 (e.g., T-shaped passage) disposed within a protruding structure 244 (e.g., generally uniform shaft). The crosswise passages 242 may include a central passage 246 extending from the interior 222 of the syringe barrel 160 to one or more lateral passages 248. As illustrated, the lateral passages 248 may be disposed in a generally tapered end 250 (e.g., conical tip portion) of the protruding structure 244. The flow control tip 162 may be configured to operate in a similar manner as discussed above with reference to the flow control tip 28.

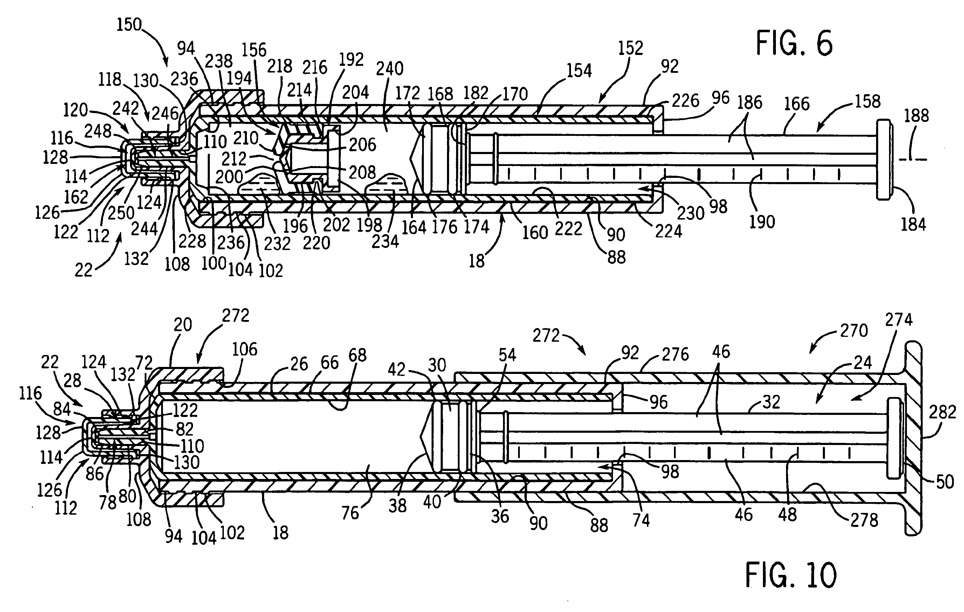

Referring generally to FIGS. 5 and 6, the syringe 154 may be retrofitted or generally assembled with the radiation shield 152 in a similar manner as described above with reference to FIGS. 1 and 2. For example, the floating plunger 156 and the primary plunger head 164 may be disposed one after the other into the interior 222 of the syringe barrel 160. However, the push rod 166 may be coupled to the primary plunger head 164 at a later stage in the assembly 150. At this point, the sleeve 18 may be disposed concentrically about the syringe barrel 160. For example, the first end 226 of the syringe barrel 160 may be inserted into the second opening 100 of the sleeve 18 until the first end 226 reaches the flange 96. Subsequently, the cover 20 may be threaded onto the sleeve 18 via engagement of threads 102 and 104. Similar to the embodiment of FIGS. 1-4, the syringe 154 may be compressively secured between the cover 20 and the sleeve 18.

Subsequently, the first substance 232 may be supplied or forced to flow through the flow control tip 28 into the first chamber 238. Alternatively, the push rod 166 may be inserted through the first opening 98 in the sleeve 18 and into the syringe barrel 160, such that the push rod 166 may be coupled with the primary plunger head 164. Then, the primary plunger 158 may be pulled outwardly from the syringe barrel 160 to create a suction that may draw the first substance 232 into the first chamber 238. Thus, the first substance 232 may be substantially or entirely shielded by the radiation shield 152 during at least most or all of the filling procedure. However, some external shielding devices, tubing, and so forth may be used to provide complete radiation shielding during the filling process. In contrast, the second substance 234, e.g., a non-radioactive substance, may be disposed within the second chamber 240 during the initial assembly process of inserting the primary plunger head 164 into the syringe barrel 160. Finally, the luer plug 22 may be secured with the end fitting 112 of the cover 20.

FIG. 6 is a cross-sectional view of an embodiment of the syringe assembly 150 having the radiation shield 152 completely assembled or retrofitted with the syringe 154. As illustrated in FIG. 6, the syringe assembly 150 is disposed in a no flow configuration similar to the embodiment discussed in detail above with reference to FIG. 3. In other words, the flow control tip 162 is sealed within the fluid outlet 114 of the end fitting 112 disposed on the cover 20. In addition, the luer plug 22 is disposed in a blocking configuration over the fluid outlet 114. The syringe assembly 150 may be reconfigured into a flow configuration by removing the luer plug 22 and partially unthreading the cover 20 from the sleeve 18.

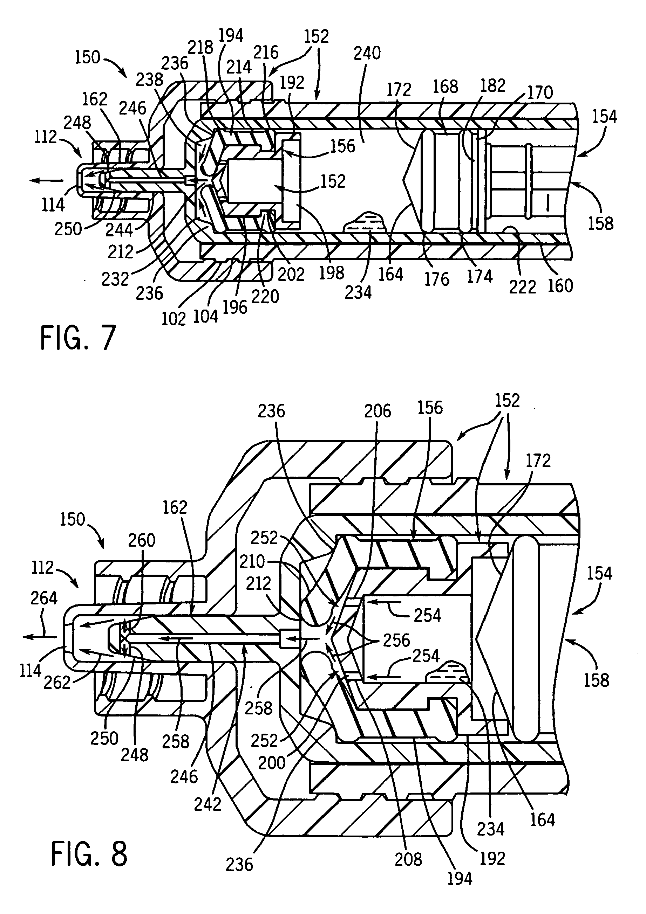

FIG. 7 is a cross-sectional view of the syringe assembly 150 of FIGS. 5 and 6, further illustrating the cover 20 partially unthreaded from the sleeve 18 without the luer plug 22 connected to the end fitting 112. In other words, the illustrated syringe assembly 150 can permit passage of the first substance 232 (e.g., a radiopharmaceutical) followed by the second substance 234 (e.g., a biocompatible flush) through the flow control tip 162 and the surrounding male luer 116 of the end fitting 112. In the illustrated embodiment, the floating plunger 156 may be abutted against the plunger stop 236 after discharging the first substance 232. The first substance 232 may be discharged from the first chamber 238 between the floating plunger 156 and the second end 228 of the syringe barrel 160 by depressing the primary plunger 158 lengthwise along the axis 188. As the primary plunger 158 moves lengthwise along the syringe barrel 160, the check valve 194 remains sealed against the fluid passage insert 192 due to the pressure differential between the first and second chambers 238 and 240. Upon reaching the plunger stop 236, the floating plunger 156 may become stationary to actuate the check valve 194.

In other words, the check valve 194 may remain closed or sealed with the fluid passage insert 192 as long as the floating plunger 156 is capable of moving in response to a pressure differential between the first and second chambers 238 and 240. As such, the movement of the floating plunger 156 maintains a fluid pressure balance between the first and second chambers 238 and 240, such that the seal is maintained by the check valve 194. When movement is no longer possible at the plunger stop 236, the force or pressure of the second substance 234 disposed in the second chamber 240 overcomes the check valve 194 to enable discharge of the second substance 234. At this stage, the primary plunger 158 moves lengthwise along the syringe barrel 160 while the floating plunger 156 remains stationary.

FIG. 8 is a partial cross-sectional view of the syringe assembly 150 of FIGS. 5-7, further illustrating actuation of the floating plunger 156 at the plunger stop 236. As illustrated, the mouth portion 210 of the check valve 194 is disposed at an offset away from the throat portion 200 of the fluid passage insert 192. In other words, a substantial gap, such as a generally conical or tapered annular gap 252, may exist between the mouth portion 210 and the throat portion 200. In this generally unrestricted configuration, the second substance 234 disposed between the primary plunger head 164 and the floating plunger 156 may be forced through the passages 206 and 208, the gap 252, the central passage 246, the lateral passages 248, the fluid cavity 136, and out through the fluid outlet 114 as illustrated by arrows 254, 256, 258, 260, 262, and 264 (respectively).

In certain embodiments, as discussed above, the second substance 234 may include a biocompatible flushing fluid, such as a heparin solution, sterilized water, a glucose solution, saline, or another suitable substance. Accordingly, the second fluid injection or discharge may substantially flush out or clean the various passages and interior portions of the syringe assembly 150. Then, the syringe assembly 150 may be resealed and shielded by rethreading the cover 20 completely back onto the sleeve 18 and reattaching the luer plug 22 with the end fitting 112. Thus, the syringe assembly 150 may be safely returned or disposed of with a substantially reduced likelihood of radiation exposure. Finally, all or a substantial portion of the radiation shield 152 may be removed from the syringe 154 for subsequent use with one or more additional syringes containing a radioactive material, such as a radiopharmaceutical.

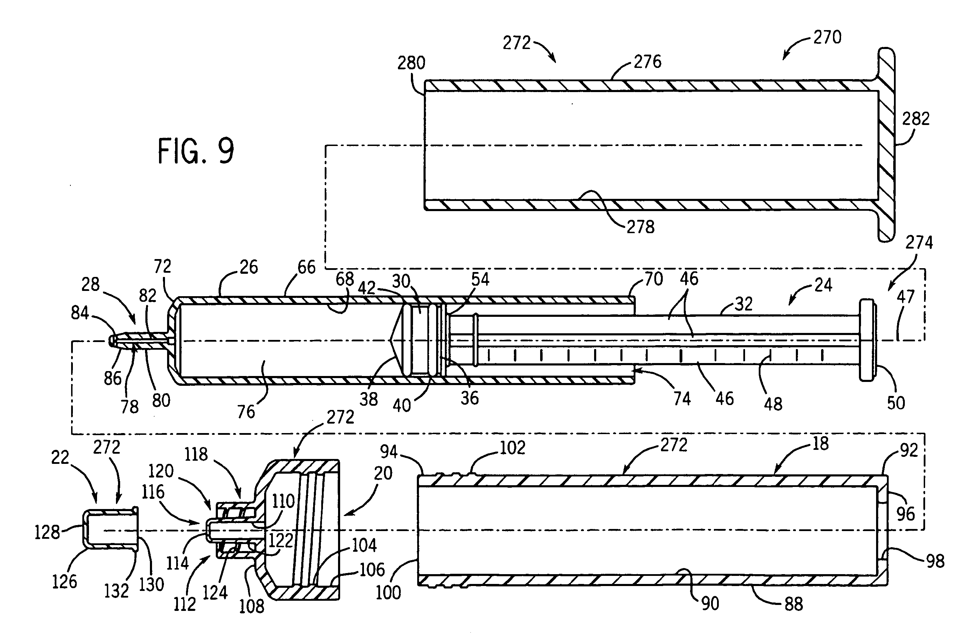

FIGS. 9 and 10 are cross-sectional views of an alternative embodiment of a radiation shielded syringe assembly 270 having a radiation shield 272 and a syringe 274. The embodiment of FIGS. 9 and 10 is similar to FIGS. 1-4 except that the radiation shield 272 may exclude the insert 16 and include a radiation shielded outer sleeve 276 (e.g., movable syringe casing). In addition, the syringe 274 may be identical to the syringe 14 of FIGS. 1-4 without the insert 16. However, in certain embodiments, the syringe assembly 270 may include the insert 16 in addition to the outer sleeve 276. Moreover, in certain embodiments, the illustrated syringe 274 may include one or more of the floating plungers 156 of FIGS. 5-8. Accordingly, the syringe 274 may be modified to include the plunger stop 236 of FIGS. 5-8.

FIG. 9 is an exploded cross-sectional view of the syringe assembly 270 illustrating the radiation shield 272 exploded from the syringe 274. As discussed above, the radiation shield 272 includes the sleeve 18, the cover 20, and the luer plug 22 as discussed above with reference to FIGS. 1-4 as well as the outer sleeve 276. The illustrate syringe 274 may include the plunger 24 having the plunger head 30 coupled to the push rod 32 with or without the intermediate insert 16. In addition, the syringe 274 may include the syringe barrel 26 having the flow control tip 28.

The syringe assembly 270 may be assembled in a similar manner as described above with reference to FIGS. 1 and 2, except that the outer sleeve 276 may be disposed about the plunger 24 and the sleeve 18 after assembling the other components. For example, the plunger head 30 may be disposed within the syringe barrel 26 without the push rod 32. The sleeve 18 may then be disposed about the syringe barrel 26, followed by threading the cover 20 onto the sleeve 18. At this point, the push rod 32 may be coupled to the plunger head 30 by inserting the push rod 32 through the first opening 98 of the sleeve 18 and into the opening 74 of the syringe barrel 26. Once inside the syringe barrel 26, the push rod 32 may be rotated to couple threadingly with the plunger head 30.

Subsequently, the outer sleeve 276 may be disposed about the plunger 24 and the sleeve 18. For example, the end member 50 of the plunger 24 and the first end 92 of the sleeve 18 may be inserted into a generally cylindrical interior 278 of the outer sleeve 276 through an open end 280. The outer sleeve 276 may be moved lengthwise about the sleeve 18 until a closed end 282 abuts the first end 92. The interior 278 may be closely dimensioned or fit about the exterior 88 of the sleeve 18, thereby reducing the likelihood of radiation exposure and providing a generally smooth engagement of the outer sleeve 276 along the sleeve 18.

Subsequently, the syringe assembly 270 may be filled or generally charged with the fluid 76, e.g., a radiopharmaceutical, by coupling a supply of the fluid 76 to the end fitting 112. After filling the syringe assembly 270, the radiation shield 272 may be completely sealed and closed to reduce the likelihood of radiation exposure. For example, the cover 20 may be fully threaded onto the sleeve 18, such that the flow control tip 28 may be closed or generally sealed within the fluid outlet 114 of the male luer 116. In addition, the luer plug 22 may be removably coupled to the end fitting 112. FIG. 10 is a cross-sectional view of an embodiment of the syringe assembly 270 of FIG. 9, further illustrating the alternative radiation shield 272 completely assembled with the syringe 274 in a closed and radiation shielded configuration. Again, the radiation shield 272 may substantially or entirely enclose, encase, house, or generally surround all sides in all directions of the syringe 274. In addition, the syringe 274 may be compressively secured between the cover 20 and the sleeve 18.

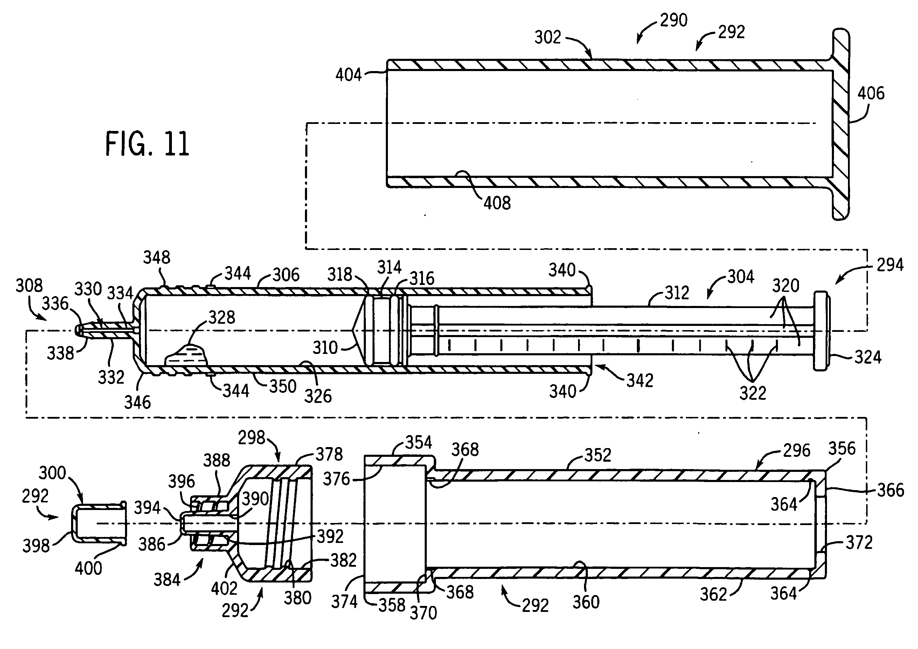

FIG. 11 is an exploded cross-sectional view of an alternative embodiment of a radiation shielded syringe 290 having a radiation shield 292 exploded from a syringe 294. In the illustrated embodiment, the radiation shield 292 may include a radiation shielded sleeve 296 (e.g., stationary syringe casing), a radiation shielded cover 298, a radiation shielded tip fitting 300 (e.g., luer plug), and a radiation shielded outer sleeve 302 (e.g., movable syringe casing). As discussed in further detail below, the radiation shielded components 296, 298, 300, and 302 of the system 292 are configured to extend substantially or entirely around the syringe 294 to provide radiation shielding during all stages of operation.

The illustrated syringe 294 may include a plunger 304 and a syringe barrel 306 having a flow control tip 308. For example, the plunger 304 may include a plunger head 310 coupled to a push rod 312. The plunger head 310 may include a cylindrical body 314 having one or more seals 316 and 318 (e.g., o-rings)..The push rod 312 may include lengthwise ribs 320, measurement indicia 322, and an end member 324. Moreover, some embodiments of the syringe 294 may include one or more floating plungers, e.g., 156 as illustrated in FIGS. 5 and 6. The plunger 304 may be disposed within the syringe barrel 306, such that the seals 316 and 318 of the plunger head 310 may be moveably sealed against an interior 326 (e.g., generally cylindrical passage). Thus, the plunger 304 may be moved inwardly and outwardly from the syringe barrel 306 to intake, discharge, or generally exchange a substance/fluid 328, such as a radioactive material or radiopharmaceutical, through the flow control tip 308.

In certain embodiments, the flow control tip 308 may include crosswise passages 330 (e.g., T-shaped passage) disposed within a protruding structure 332. For example, the crosswise passages 330 may include a central passage 334 and one or more lateral passages 336. The lateral passages 336 may be disposed in a generally tapered end 338 (e.g., conical tip portion) of the protruding structure 332. As discussed in further detail below, the flow control tip 308 may cooperate with a portion of the cover 298 to reduce the likelihood of fluid spillage and radiation exposure.

The syringe 294 may include one or more features configured to mate with the radiation shielded components 296 and 298 of the radiation shield 292. For example, the syringe barrel 306 may include one or more tool free fastening mechanisms, such as opposite snap-fit members 340 (e.g., latches) disposed at or near a first end 342 (e.g., inlet) of the syringe barrel 306. In addition, the syringe barrel 306 may include one or more guides or securement members, such as opposite protruding portions 344 (e.g., rotational locks) disposed between the first end 342 and a second end 346 (e.g., outlet) of the syringe barrel 306. For example, the rotational locks 344 may be disposed adjacent external threads 348 along a generally cylindrical exterior 350 of the syringe barrel 306. These features 340, 344, and 348 on the exterior 350 of the syringe barrel 306 may be configured to mate with corresponding features within the sleeve 296 and the cover 298.

For example, the sleeve 296 may include a main casing portion 352 and an enlarged casing portion 354 - (e.g., female fitting), wherein the main casing portion 352 may extend along a substantial portion of the length of the sleeve 296 between first and second ends 356 and 358. The main casing portion 352 may include an interior 360 (e.g., syringe receptacle) and a generally cylindrical exterior 362. In addition, the interior 360 may include one or more snap-fit/latch receptacles, such as an annular latch slot 364 disposed at or near a flange 366 at the first end 356. The interior 360 may include one or more guide securement receptacles, such as rotational lock receptacles 368 (e.g., opposite grooves) at an interface 370 (e.g., stepped portion) between the main casing portion 352 and the enlarged casing portion 354. The sleeve 296 may include a first opening 372 (e.g., plunger passageway) in the flange 366. At the second end 358, the enlarged casing portion 354 may include a second opening 374 having a generally cylindrical interior 376, which may be configured to extend around a generally cylindrical exterior 378 of the cover 298. As discussed in further detail below, the sleeve 296 and the cover 298 may be moved lengthwise with respect to one another as the syringe barrel 306 threadingly engages or disengages with the cover 298. For example, the cover 298 may include internal threads 380 disposed along an interior surface 382 (e.g., cylindrical receptacle), which may receive the external threads 348 and corresponding exterior 350 of the syringe barrel 306.

The cover 298 may include an external fluid coupling 384 (e.g., end fitting) having a male portion 386 (e.g., male luer) and a supplemental securement portion 388 (e.g., luer collar). The illustrated male luer 386 may include a central passage 390, a compression fitting 392 (e.g., tapered surface), and a fluid outlet 394. In addition, the luer collar 388 may include internal threads 396 facing the compression fitting 392 of the male luer 386. In certain embodiments, the flow control tip 308 of the syringe 294 may seal and unseal against the fluid outlet 394 as the cover 298 threads onto or off of the external threads 348 of the syringe barrel 306. In addition, the luer plug 300 may be coupled with the end fitting 384. In certain embodiments, the luer plug 300 may include a cup shaped body 398 having opposite tabs 400 (e.g., external luer threads), which may be coupled with the internal threads 396 of the luer collar 388.

As illustrated in FIG. 11, the syringe 294 may be assembled or retrofitted with the radiation shield 292 as follows. For example, the plunger head 310 may be disposed within the syringe barrel 306 separate from the push rod 312. The syringe barrel 306 may be inserted lengthwise into the sleeve 296. For example, the first end 342 of the syringe barrel 306 may be inserted into the second opening 374 of the sleeve 296, and then may be moved lengthwise along the interior 360 until the rotational locks 344 reach the interface 370. Then, the syringe barrel 306 may be rotated to align the rotational locks 344 with the corresponding lock receptacles 368 in the main casing portion 352. Once aligned, the rotational locks 344 may be moved lengthwise into the lock receptacles 368 in the main casing portion 352. During this movement, the latches 340 disposed on the first end 342 of the syringe barrel 306 may be moved into engagement with the latch slot 364 disposed at or near the flange 366 of the main casing portion 352. Eventually, the latches 340 removably snap into place with the latch slot 364 to hold the lengthwise position of the syringe barrel 306 within the interior 360 of the sleeve 296. In addition, the engagement between the rotational locks 344 and the corresponding lock receptacles 368 may hold the rotational position of the syringe barrel 306 with respect to the sleeve 296.

Subsequently, the cover 298 may be disposed about the flow control tip 308 and the second end 346 of the syringe barrel 306 until the internal threads 380 engage with the external threads 348. During this movement, the exterior 378 of the cover 298 may extend lengthwise into the second opening 374 of the enlarged casing portion 354 of the sleeve 296. After engaging the external and internal threads 348 and 380, the cover 298 may be threaded rotatingly onto the syringe barrel 306 until the syringe barrel 306 is generally compressed between the flange 366 of the sleeve 296 and a closed front 402 of the cover 298. At this point, the tapered end 338 of the flow control tip 308 may be substantially or entirely blocked/sealed within the fluid outlet 394 of the male luer 386.

In addition, the luer plug 300 may be coupled to the luer collar 396 of the end fitting 384. Subsequently, the push rod 312 may be extended through the first opening 372 of the sleeve 296 and through the first end 342 of the syringe barrel 306. The push rod 312 may then couple with the plunger head 310, for example, by rotatingly threading the push rod 312 with respect to the plunger head 310 as discussed in detail above. Finally, the outer sleeve 302 may be disposed about the plunger 304 and the surrounding sleeve 296. For example, the peripheral portion of the plunger 304 and the first end 356 of the sleeve 296 may be inserted lengthwise into an open end 404 of the outer sleeve 302 until the end member 324 of the plunger 304 abuts a closed end 406 of the outer sleeve 302. In addition, the exterior 362 of the sleeve 296 may generally slide into a generally cylindrical interior 408 of the outer sleeve 302.

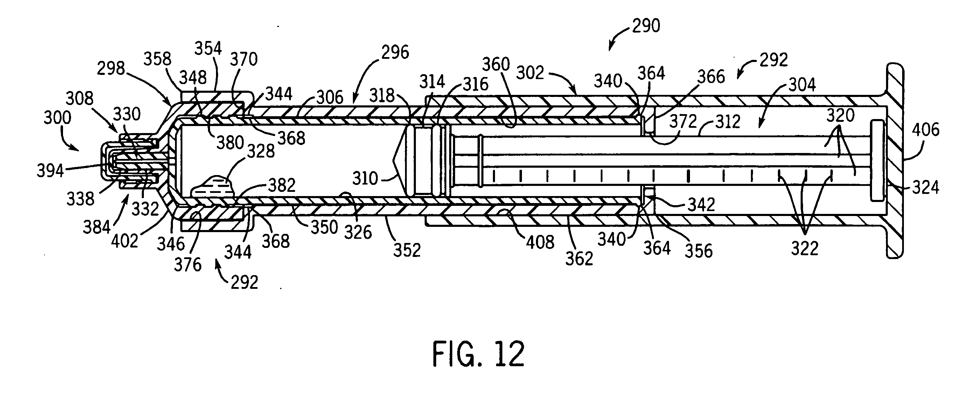

FIG. 12 is a cross-sectional view of an embodiment of the radiation shielded syringe 290 having the syringe 294 assembled or retrofitted with the radiation shield 292. In the illustrated configuration, a user may be substantially or entirely shielded from radiation originating in the substance or fluid 328 disposed within the syringe 294 as the radiation shielded components 296, 298, 300, and 302 extend substantially or entirely around all sides of the syringe 294. Moreover, the flow control tip 308 may be substantially or entirely sealed within the male luer 386 of the end fitting 384. Specifically, the tapered end 338 of the protruding structure 332 is disposed at least against, into, or partially or entirely through the fluid outlet 394, such that the lateral passages 336 may be generally closed off or sealed within the central passage 390. In addition, the syringe 294 may be compressively secured between the cover 298 and the sleeve 296.

The syringe 294 may be operated by removing the luer plug 300, unsealing the flow control tip 308, coupling the end fitting 384 to a suitable fluid delivery or transmission system, and moving the outer sleeve 302 along with the plunger 304 disposed inside. As such, the substance/fluid 328 may be input, output, or generally exchanged with the syringe 294. Again, similar to the embodiment of FIGS. 1-4, the flow control tip 308 may be unsealed by partially unthreading the cover 298 from the syringe barrel 306, thereby freeing the tapered end 338 from the fluid outlet 394. As a user moves the outer sleeve 302 lengthwise along the exterior 362 of the sleeve 296, the closed end 406 biases the end member 324 of the plunger 304 in the desired direction to move the substance/fluid 328 disposed within the syringe barrel 306. The closed end 406 of the outer sleeve 302 may include a fastener to removably attach the end member 324 of the plunger 304. In certain embodiments, an adhesive tape, pad, glue, or other material may be disposed between the end member 324 and the interior of the closed end 406. In other embodiments, a snap-fit mechanism or mating threads may be disposed between the end member 324 and the interior of the closed end 406.

During operation of the radiation shielded syringe 290, the radiation shield 292 may provide at least substantial or complete radiation shielding in generally all directions and all sides around the syringe 294 up to the fluid outlet 394 of the cover 298. In addition, radiation shielded connectors, tubing, and other devices may be coupled to the end fitting 384 to provide additional shielding downstream of the radiation shielded syringe 290. As discussed above, after a particular use of the radiation shielded syringe 290, the various radiation shielded components 296, 298, 300, and 302 may be removed, cleaned, and generally reused on one or more additional syringes.

In certain embodiments, the syringes illustrated and described above with reference to FIGS. 1-12 may be filled or pre-filled with one or more medical fluids, such as contrast agents, radiopharmaceuticals, tagging agents, biocompatible flushes, or combinations thereof. For example, the disclosed radiation shielded syringes, e.g., 10, 150, 270, and 290, may be filled or pre-filled with a first medical fluid in a first chamber and a second medical fluid in a second chamber. The first medical fluid may include a radioisotope or radiopharmaceutical for radiation-based treatment or medical imaging, such as positron emission tomography (PET) or single photon emission computed tomography (SPECT). In addition, the second medical fluid may include a biocompatible flush, such as heparin solution, sterilized water, glucose solution, saline, or another suitable substance. The disclosed radiation shielded syringes may be used to inject the first and second medical fluids one after another into a subject or patient. Alternatively, the disclosed radiation shielded syringes may be filled or pre-filled with a single medical fluid, such as a radiopharmaceutical. In certain embodiments, the subject may be scanned or generally imaged by a suitable medical diagnostic and/or imaging system, such as listed above. For example, after the radiopharmaceutical enters the blood stream and focuses on a particular organ or area of interest, the diagnostic and/or imaging system may function to acquire imaging data, process the data, and output one or more images. Thus, the diagnostic and/or imaging system may include detector/acquisition hardware and software, data/image processing hardware and software, data/image storage hardware and software, a display, a printer, a keyboard, a mouse, a computer workstation, a network, and other associated equipment.

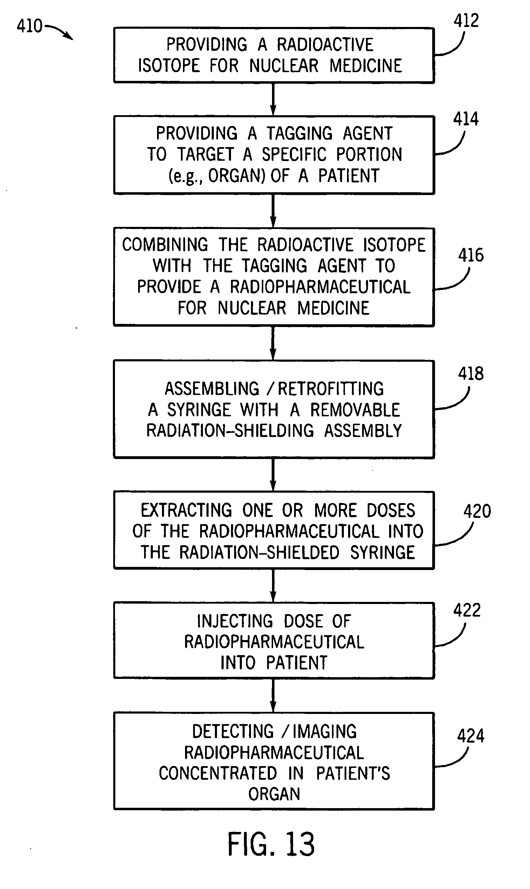

FIG. 13 is a flowchart illustrating an exemplary nuclear medicine process utilizing the radiation shielded syringes, e.g., 10, 150, 270, and 290, as illustrated with reference to FIGS. 1-12. As illustrated, the process 410 begins by providing a radioactive isotope for nuclear medicine at block 412. For example, block 412 may include eluting technetium-99m from a radioisotope generator as discussed in further detail below. At block 414, the process 410 proceeds by providing a tagging agent (e.g., an epitope or other appropriate biological directing moiety) adapted to target the radioisotope for a specific portion, e.g., an organ, of a patient. At block 416, the process 410 then proceeds by combining the radioactive isotope with the tagging agent to provide a radiopharmaceutical for nuclear medicine. In certain embodiments, the radioactive isotope may have natural tendencies to concentrate toward a particular organ or tissue and, thus, the radioactive isotope may be characterized as a radiopharmaceutical without adding any supplemental tagging agent. At block 418, the process 410 may then involve assembling or retrofitting a syringe with a removable radiation shielding assembly, e.g., 12, 152, 272, or 292, as discussed in detail above. At block 420, the process 410 then may proceed by extracting one or more doses of the radiopharmaceutical into the radiation shielded syringe, e.g., 10, 150, 270, or 290, which may be suitable for administering the radiopharmaceutical to a patient in a nuclear medicine facility or hospital. At block 422, the process 410 proceeds by injecting or generally administering a dose of the radiopharmaceutical into a patient. After a pre-selected time, the process 410 proceeds by detecting/imaging the radiopharmaceutical tagged to the patient's organ or tissue (block 424). For example, block 424 may include using a gamma camera or other radiographic imaging device to detect the radiopharmaceutical disposed on or in or bound to tissue of a brain, a heart, a liver, a tumor, a cancerous tissue, or various other organs or diseased tissue.

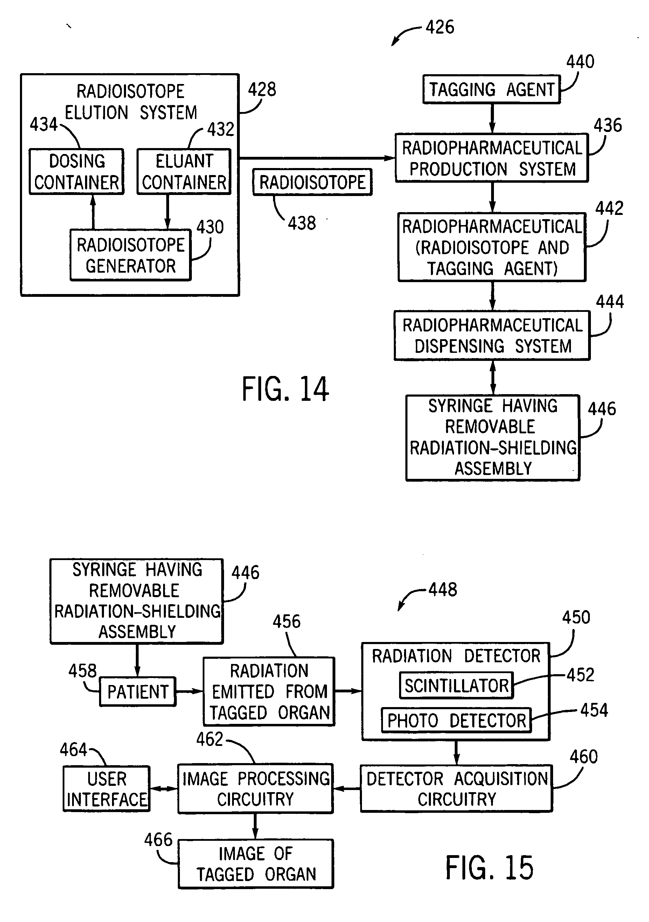

FIG. 14 is a block diagram of an exemplary system 426 for providing a radiation shielded syringe, e.g., 10, 150, 270, and 290, having one or more medical fluids (e.g., radiopharmaceutical and biocompatible flush) for use in a nuclear medicine application. As illustrated, the system 426 may include a radioisotope elution system 428 having a radioisotope generator 430, an eluant supply container 432, and an eluate output container or dosing container 434. In certain embodiments, the eluate output container 434 may be in vacuum, such that the pressure differential between the eluant supply container 432 and the eluate output container 434 facilitates circulation of an eluant (e.g., saline) through the radioisotope generator 430 and out through an eluate conduit into the eluate output container 434. As the eluant, e.g., a saline solution, circulates through the radioisotope generator 430, the circulating eluant generally washes out or elutes a radioisotope, e.g., Technetium-99m. For example, one embodiment of the radioisotope generator 430 includes a radiation shielded outer casing (e.g., lead shell) that encloses a radioactive parent, such as molybdenum-99, adsorbed to the surfaces of beads of alumina or a resin exchange column. Inside the radioisotope generator 430, the parent molybdenum-99 transforms, with a half-life of about 67 hours, into metastable technetium-99m. The daughter radioisotope, e.g., technetium-99m, is generally held less tightly than the parent radioisotope, e.g., molybdenum-99, within the radioisotope generator 430. Accordingly, the daughter radioisotope, e.g., technetium-99m, can be extracted or washed out with a suitable eluant, such as an oxidant-free physiologic saline solution. The eluate output from the radioisotope generator 430 into the eluate output container 434 generally includes the eluant and the washed out or eluted radioisotope from within the radioisotope generator 430. Upon receiving the desired amount of eluate within the eluate output container 434, a valve may be closed to stop the eluant circulation and output of eluate. As discussed in further detail below, the extracted daughter radioisotope can then, if desired, be combined with a tagging agent to facilitate diagnosis or treatment of a patient (e.g., in a nuclear medicine facility).

As further illustrated in FIG. 14, the system 426 includes a radiopharmaceutical production system 436, which functions to combine a radioisotope 438 (e.g., technetium-99m solution acquired through use of the radioisotope elution system 428) with a tagging agent 440. In some embodiment, this radiopharmaceutical production system 436 may refer to or include what are known in the art as "kits" (e.g., Technescan® kit for preparation of a diagnostic radiopharmaceutical). Again, the tagging agent may include a variety of substances that are attracted to or targeted for a particular portion (e.g., organ, tissue, tumor, cancer, etc.) of the patient. As a result, the radiopharmaceutical production system 436 produces or may be utilized to produce a radiopharmaceutical including the radioisotope 438 and the tagging agent 440, as indicated by block 442. The illustrated system 426 may Include a radiopharmaceutical dispensing system 444, which facilitates extraction of the radiopharmaceutical into a vial or syringe 446. In the illustrated embodiment, the syringe may be a radiation shielded syringe as illustrated and described above with reference to FIGS. 1-12. Thus, the system 426 may fill the syringe with an additional substance or medical fluid, such as a biocompatible flush. For example, the syringe assembly 150 of FIGS. 5-8 may be filled with a radiopharmaceutical and a biocompatible flush in sequential chambers separated by the floating plunger 156. In certain embodiments, the various components and functions of the system 426 are disposed within a radiopharmacy, which prepares the syringe 446 of the radiopharmaceutical for use in a nuclear medicine application. For example, the syringe 446 may be prepared and delivered to a medical facility for use in diagnosis or treatment of a patient.

FIG. 15 is a block diagram of an exemplary nuclear medicine imaging system 448 utilizing the radiation shielded syringe 446 of radiopharmaceutical provided using the system 426 of FIG.14. As illustrated, the nuclear medicine imagining system 448 includes a radiation detector 450 having a scintillator 452 and a photo detector 454. In response to radiation 456 emitted from a tagged organ within a patient 458, the scintillator 452 emits light that is sensed and converted to electronic signals by the photo detector 454. Although not illustrated, the imaging system 448 can include a collimator to collimate the radiation 456 directed toward the radiation detector 450. The illustrated imaging system 448 includes detector acquisition circuitry 460 and image processing circuitry 462. The detector acquisition circuitry 460 generally controls the acquisition of electronic signals from the radiation detector 450. The image processing circuitry 462 may be employed to process the electronic signals, execute examination protocols, and so forth. The illustrated imaging system 448 includes a user interface 464 to facilitate user interaction with the image processing circuitry 462 and other components of the imaging system 448. As a result, the imaging system 448 produces an image 466 of the tagged organ within the patient 458. Again, the foregoing procedures and resulting image 466 directly benefit from the one or more medical fluids (e.g., radiopharmaceutical) administered with the radiation shielded syringe as illustrated and described with reference to FIGS. 1-12.

When introducing elements of various embodiments of the present invention, the articles "a", "an", "the", and "said" are intended to mean that there are one or more of the elements. The terms "comprising", "including", and "having" are intended to be inclusive and mean that there may be additional elements other than the listed elements. Moreover, the use of "top", "bottom", "above", "below" and variations of these terms is made for convenience, but does not require any particular orientation of the components.

While the invention may be susceptible to various modifications and alternative forms, specific embodiments have been shown by way of example in the figures and have been described in detail herein. However, it should be understood that the invention is not intended to be limited to the particular forms disclosed. Rather, the invention is to cover all modifications, equivalents, and alternatives falling within the scope of the invention as defined by the following appended claims.

## Claims

1. A syringe radiation shield comprising a sleeve (18) comprising a receptacle for sliding insertion of a syringe barrel, and a cover (20) for the distal end of said syringe barrel, said cover having a substantially closed front extending to a through passage (110), and said sleeve and cover comprising radiation shielding **characterized in that** said cover comprises a receptacle for said distal end, said cover being adapted for removable coupling with said sleeve (18).

2. The shield of claim 1, further comprising a fitting (22) extending across said through passage (110) when in a coupled position with the cover (20), wherein said fitting comprises radiation shielding.

3. The shield of claim 1 or claim 2, further comprising a plunger insert (16) movably positioned within the sleeve (18), wherein said insert (16) comprises radiation shielding.

4. The shield of claim 3, wherein said insert (16) comprises one of a fastener for the head of a syringe plunger, a fastener for the shaft of a syringe plunger, and a combination fastener for the head and shaft of a syringe plunger.

5. The shield of any preceding claim, further comprising a luer fitting (112) on said cover (20).

6. A syringe according to any preceding claim, wherein said sleeve (18) includes a radially inwardly directed flange (96) at the proximal end thereof, for abutment with a syringe.

7. The shield of any preceding claim, wherein the sleeve (18), the cover (20) and passage (110) are generally concentric with one another.

8. The shield of any preceding claim, wherein the sleeve (18) comprises first threads and the cover (20) comprises second threads that mate with the first threads.

9. The shield of any preceding claim, further comprising an outer sleeve (276) that movably couples with the sleeve (18), wherein both sleeves comprise radiation shielding, and said outer shield comprises a receptacle for a syringe plunger.

10. The syringe radiation shield of any preceding claim, in combination with a syringe disposed therein.

11. The combination of claim 10, further comprising a flow control mechanism for the tip (28) of said syringe.

12. The combination of claim 11, wherein the flow control mechanism comprises a fluid outlet (114) of said passage (110) and an axially movable fastening mechanism between said sleeve (18) and said cover (20).

13. The combination of claim 12, wherein said tip (28) is disposed in said passage (110).

14. The combination of claim 13, wherein the flow control tip (28) is sealed and unsealed with said fluid outlet (114) via relative movement of said movable fastening mechanism.

15. The combination of claims 12-14, wherein the movable fastening mechanism comprises mating threads between the sleeve (18) and the cover (20).

16. The combination of any of claims 10-15, and further comprising a radiopharmaceutical disposed within the syringe.

## Patentansprüche

1. Injektionsspritzen-Strahlungsschirm mit einer Hülse (18), die einen Aüfnahmebehälter zum gleitenden Einführen eines Injektionsspritzenzylinders umfasst, und einer Abdeckung (20) für das distale Ende des Injektionsspritzenzylinders, wobei die Abdeckung eine im Wesentlichen geschlossene Vorderseite aufweist, die sich zu einer Durchführung (110) erstreckt, und wobei die Hülse und die Abdeckung eine Strahlungsabschirmung umfassen, **dadurch gekennzeichnet, dass** die Abdeckung einen Aufnahmebehälter für das distale Ende aufweist, wobei die Abdeckung für eine lösbare Verbindung mit der Hülse (18) ausgelegt ist.

2. Schirm nach Anspruch 1, ferner umfassend ein Anschlussstück (22), das sich quer über die Durchführung (110) erstreckt, wenn es sich in einem mit der Abdeckung (20) verbundenem Zustand befindet, wobei das Anschlusstück eine Strahlungsabschirmung umfasst.

3. Schirm nach Anspruch 1 oder Anspruch 2, ferner umfassend einen Kolbeneinsatz (16), der innerhalb der Hülse (18) beweglich positionierbar ist, wobei der Einsatz (16) eine Strahlungsabschirmung umfasst.

4. Schirm nach Anspruch 3, wobei der Einsatz (16) eines von einem Befestigungselement für den Kopf eines Injektionsspritzenkolbens, ein Befestigungselement für den Schaft eines Injektionsspritzenkolbens und ein Kombinationsbefestigungselement für den Kopf und den Schaft eines Injektionsspritzenkolbens umfasst.

5. Schirm nach einem der vorherigen Ansprüche, ferner umfassend ein Luer-Anschlussstück (112) an der Abdeckung (20).

6. Schirm nach einem der vorherigen Ansprüche, wobei die Hülse (18) einen radial nach innen gerichteten Flansch (96) an ihrem proximalan Ende zur Anlage gegen eine Injektionsspritze aufweist.

7. Schirm nach einem der vorherigen Ansprüche, wobei die Hülse (18), die Abdeckung (20) und die Durchführung (110) allgemein konzentrisch zueinander sind.

8. Schirm nach einem der vorherigen Ansprüche, wobei die Hülse (18) ein erstes Gewind umfasst und die Abdeckung ein zu dem ersten Gewinde passendes zweites Gewinde umfasst.

9. Schirm nach einem der vorherigen Ansprüche, ferner umfassend eine außere Hülse (276), die lösbar mit der Hülse (18) verbunden ist, wobei beide Hülsen eine Strahlungsabschirmung umfassen und die äußere Hülse einen Aufnahmebehälter für einen Injektionsspritzenkolben umfasst.

10. Injektionsspritzen-Strahlungsschirm nach einem der vorherigen Ansprüche, in Kombination mit einer dann angeordneten Injektionsspritze.

11. Kombination von Anspruch 10, ferner umfassend einen Strömungskontrollmechanismus für die Spitze (28) der injektionsspritze.

12. Kombination von Anspruch 11, wobei der Stromungskontrollmechanismus einen Fluidausfluss (114) der Durchführung (110) und einen axial bewegbaren Befestigungsmechanismus zwischen der Hülse (18) und der Abdeckung (20) umfasst.

13. Kombination von Anspruch 12, wobei die Spitze (28) in der Durchführung (110) angeordnet ist.

14. Kombination von Anspruch 13, wobei Strömungskontrollspitze (28) über eine Relativbewegung des beweglichen Befestigungsmechanismus verschlossen und geöffnet wird.

15. Kombination der Ansprüche 12-14, wobei der bewegliche Befestigungsmechanismus passende Gewinde zwischen der Hülse (18) und der Abdeckung (20) umfasst.

16. Kombination von einem der Ansprüche 10-15, und ferner umfassend ein Radiopharmazeutikum, das innerhalb der Injektionsspritze angeordnet ist.

## Revendications

1. Blindage de seringue contre les rayonnements comprenant un manchon (18) comprenant un réceptacle pour l'insertion coulissante d'un cylindre de seringue, et un cache (20) pour l'extrémité distale dudit cylindre de seringue (110), ledit cache ayant un devant fermé s'étendant au travers d'un passage traversant (110), ledit manchon et ledit cache comprenant un blindage contre les rayonnements **caractérisé en ce que** ledit cache comprend un réceptacle pour ladite extrémité distale, ledit cache étant adapté pour s'accoupler de façon amovible avec ledit manchon (18).

2. Blindage selon la revendication 1, comprenant en outre une fixation (22) s'étendant à travers ledit passage traversant (110) lorsqu'elle est dans une position couplée avec le cache (20), dans lequel ladite fixation comprend un blindage contre les rayonnements.

3. Blindage selon la revendication 1 ou la revendication 2, comprenant en outre un insert de type piston (16) positionné de façon mobile à l'intérieur du manchon (18), dans lequel ledit insert (16) comprend un blindage contre les rayonnements.

4. Blindage selon la revendication 3, dans lequel ledit insert (16) comprend une d'une attache pour la tête d'un piston de seringue, d'une attache pour la tige d'un piston de seringue et d'une attache combinée pour la tête et la tige d'un piston de seringue.

5. Blindage selon l'une quelconque des revendications précédentes, comprenant en outre un raccord Luer (112) sur ledit cache (20).

6. Seringue selon l'une quelconque des revendications précédentes, dans laquelle ledit manchon (18) comprend un rebord dirigé radialement vers l'intérieur (96) à son extrémité proximale, pour mise en butée avec une seringue.

7. Blindage selon l'une quelconque des revendications précédentes, dans lequel le manchon (18), le cache (20) et le passage (110) sont généralement concentriques les uns par rapport aux autres.

8. Blindage selon l'une quelconque des revendications précédentes, dans lequel le manchon (18) comprend des premiers filets et le cache (20) comprend des seconds filets qui s'accouplent avec les premiers filets.

9. Blindage selon l'une quelconque des revendications précédentes, comprenant en outre un manchon externe (276) qui s'accouple de façon mobile avec le manchon (18), dans lequel les deux manchons comprennent un blindage contre les rayonnements, et ledit blindage extérieur comprend un réceptacle pour un piston de seringue.

10. Blindage de seringue contre les rayonnements selon l'une quelconque des revendications précédentes, en combinaison avec une seringue disposée à l'intérieur de celui-ci.

11. Combinaison selon la revendication 10, comprenant en outre un mécanisme de régulation de débit pour la pointe (28) de ladite seringue.

12. Combinaison selon la revendication 11, dans laquelle le mécanisme de régulation de débit comprend une sortie de fluide (114) dudit passage (110) et un mécanisme de fixation mobile axialement entre ledit manchon (18) et ledit cache (20).

13. Combinaison selon la revendication 12, dans laquelle ladite pointe (28) est disposée dans ledit passage (110).

14. Combinaison selon la revendication 13, dans laquelle la pointe de régulation de débit (28) est scellée et descellée avec ledit orifice de sortie de fluide (114) via le mouvement relatif dudit mécanisme de fixation mobile.

15. Combinaison selon les revendications 12 à 14, dans laquelle le mécanisme de fixation mobile comprend des filets accouplés entre le manchon (18) et le cache (20).

16. Combinaison selon l'une quelconque des revendications 10 à 15, comprenant en outre un produit radiopharmaceutique disposé à l'intérieur de la seringue.
